# EUROPEAN PATENT APPLICATION

(11) **EP 4 316 483 A1**
(43) Date of publication of application: **07.02.2024**
(21) Application number: 22776079.0
(22) Date of filing: 23.03.2022
(51) Int. Cl.: A61K 31/198, A61P 43/00, A61P 1/00, A61P 19/00, A23L 33/175

(54) **COMPOSITION FOR ENHANCING PHYSIOLOGICAL EFFICACY OF LACTIC ACID BACTERIA**

(30) Priority: 26.03.2021 KR 20210039472
(71) Applicant: Seoul National University R & DB Foundation, Seoul 08826 (KR)
(72) Inventor: LEE, Won Jae, Seoul 04387 (KR); KIM, Eun Kyoung, Seoul 08730 (KR); KIM, Sung Hee, Seoul 08729 (KR); LEE, Kyung Ah, Seoul 06937 (KR); KIM, Bo Ram, Gyeonggi-do 13389 (KR)
(74) Representative: Schön, Christoph
(86) International application number: PCT/KR2022/004044
(87) International publication number: WO 2022/203379

(57) **Abstract**

The present invention relates to a composition for enhancing efficacy of lactic acid bacteria. More specifically, the composition includes at least two branched-chain amino acids selected from the group consisting of leucine, isoleucine and valine, thereby enhancing physiological activity of lactic acid bacteria, for example, the growth promoting efficacy or the intestinal damage inhibitory efficacy of lactic acid bacteria.

## Description

### [Technical Field]

The present invention relates to a composition for enhancing physiological efficacy of lactic acid bacteria.

### [Background Art]

Lactic acid bacteria are bacteria that produce lactic acid using sugar as an energy source, and are found in the digestive organs, oral cavity, etc. of humans or mammals and widely distributed in nature such as diverse fermented food products. Lactic acid bacteria is one of the microorganisms that have been widely used for a long time by mankind, and has good function of preventing decay in the intestines while not generating substances harmful to the intestines of humans or animals.

As the representative probiotics, lactic acid bacteria not only have antibacterial effect, but also regulate intestinal microflora of the host so as to exhibit beneficial effects in various aspects such as inhibition of various intestinal diseases and enhancing immunity. Therefore, it is increasing attention to development of materials for various foods. Such lactic acid bacteria are widely used in human life from fermented milk products to various fermented foods, sauces, beverages, pharmaceuticals, and feed additives for livestock.

Meanwhile, lactic acid bacteria products use prebiotic additives such as vitamins and dietary fiber together for the purpose of increasing the effect of lactic acid bacteria. Conventional or current lactic acid bacteria-related products are only simple combinations of several ingredients and do not show great efficacy or, if taken in a large dose, problems such as adversely affecting *in vivo* metabolism of the user may occur. Further, there is a lack of research on how the additives influence on the physiological activity of lactic acid bacteria.

### [Summary of Invention]

### [Problems to be Solved by Invention]

An object of the present invention is to provide a composition for enhancing the efficacy of lactic acid bacteria.

In addition, another object of the present invention is to provide a pharmaceutical composition for promoting growth or inhibiting intestinal damage.

Further, another object of the present invention is to provide a food composition for promoting growth or inhibiting intestinal damage.

### [Means for Solving Problems]

1. A composition for enhancing efficacy of lactic acid bacteria, including: at least two branched-chain amino acids (BCAAs) selected from the group consisting of leucine, isoleucine and valine.
2. The composition according to the above 1, including leucine, isoleucine and valine.
3. The composition according to the above 1, wherein the efficacy of lactic acid bacteria is to promote the growth of a subject or to inhibit intestinal damage.
4. The composition according to the above 1, wherein the efficacy of lactic acid bacteria is to increase at least one of body weight or bone density of a nutrient-deficient subject, or to inhibit leaky gut of the nutrient-deficient subject.
5. The composition according to the above 1, wherein the lactic acid bacteria is at least one selected from the group consisting of *Lactobacillus spp., Lactococcus spp., Enterococcus spp., Streptococcus spp.* and *Bififobacterium spp.*
6. The composition according to the above 5, wherein the *Lactobacillus spp.* is at least one selected from the group consisting of: *Lactiplantibacillus plantarum; Lactobacillus paracasei; Lactobacillus rhamnosus; Lactobacillus fermentum; Lactobacillus casei; Lactobacillus acidophilus; Lactobacillus gasseri; Lactobacillus delbrueckii subsp. Bulgaricus; Lactobacillus reuteri; Lactobacillus helveticus;* and *Lactobacillus salivarius.*
7. A pharmaceutical composition for promoting growth or inhibiting intestinal damage, including: the composition according to any one of the above 1 to 6; and lactic acid bacteria.
8. The pharmaceutical composition according to the above 7, wherein the lactic acid bacteria is at least one selected from the group consisting of: *Lactiplantibacillus plantarum; Lactobacillus paracasei; Lactobacillus rhamnosus; Lactobacillus fermentum; Lactobacillus casei; Lactobacillus acidophilus; Lactobacillus gasseri; Lactobacillus delbrueckii subsp. Bulgaricus; Lactobacillus reuteri; Lactobacillus helveticus;* and *Lactobacillus salivarius.*
9. A pharmaceutical composition for preventing or treating at least one disease selected from the group consisting of growth disorders, hypogrowth, osteoporosis, osteomalacia, osteopenia, environmental enteropathy and leaky gut syndrome, including: the composition according to any one of the above 1 to 6; and lactic acid bacteria.
10. A food composition for promoting growth or inhibiting intestinal damage, including: the composition according to any one of the above 1 to 6; and lactic acid bacteria.

### [Advantageous effects]

The composition for enhancing the efficacy of lactic acid bacteria of the present invention can enhance the physiologically active function of lactic acid bacteria, for example, the efficacy of lactic acid bacteria to promote the growth of a subject or to inhibit intestinal damage by including the branched-chain amino acids.

The composition for enhancing the efficacy of lactic acid bacteria of the present invention can improve the physiologically active function of lactic acid bacteria as a result of including the branched-chain amino acids synthesized by a synthase of the branched-chain amino acids, which lacked in lactic acid bacteria.

The pharmaceutical or food composition of the present invention can provide growth promoting effects and/or intestinal damage improving effects in the subject as a result of including the composition for enhancing the efficacy of lactic acid bacteria and the lactic acid bacteria.

### [Brief Description of Drawings]

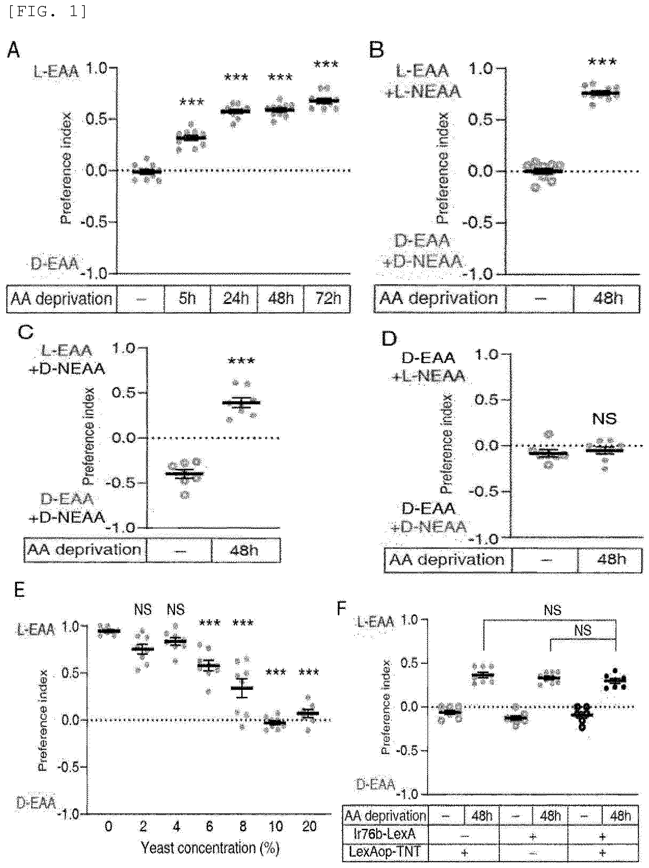

FIG. 1 shows results of observing nutritional satisfaction through analysis of the intake behavior of fruit flies (Drosophila) induced by protein deficiency. Specifically, FIG. 1A shows, after placing Drosophila in a protein-containing medium and a protein-deficient medium by time, respectively, results of confirming which food is preferred among L-form essential amino acid (a form of amino acid existing in nature, L-form essential amino acid, L-EAA) and D-form essential amino acid (optical isomer, that is, enantiomer of L-form essential amino acid, D-EAA). It was confirmed that the longer the exposure time to the protein-deficient food, the higher the preference index for L-EAA. FIG. 1B shows results of the intake preference of Drosophila maintained for 48 hours in the protein-deficient medium. It was confirmed that it acts in the direction to satisfy L-form amino acid, which is the form of amino acid existing in nature. FIG. 1C shows results of confirming that Drosophila prefers the intake of L-form essential amino acids among L-form amino acids to supplement for protein deficiency. FIG. 1D shows results of confirming that the intake preference for L-form non-essential amino acid (L-form Nonessential amino acid, L-NEAA) is not increased even in a protein-deficient situation. FIG. 1E shows results of confirming the L-EAA intake preference of Drosophila that ate a medium composed of different ratios of protein to the same calorific value. The intake preference for L-EAA was decreased as the protein content of the ingested food was increased. FIG. 1F shows results of confirming that, even if the activity of Ir76b cells, known as a taste receptor that recognizes a protein, is inhibited by inducing overexpression of tetanus toxin (TNT), it does not affect the L-EAA intake preference of Drosophila in the protein-deficient situation. This suggests that the essential amino acid intake behavior to satisfy protein deficiency is regulated through pathways other than the previously known signaling pathways. AA deprivation means an amino acid deficiency, L-EAA means an essential amino acid in the L-form naturally occurring, D-EAA means an essential amino acid in the enantiomer form, and L-NEAA means a non-essential amino acid. Further, in the case of Ir76b-LexA, fruit flies expressing LexA protein at the Ir76b expression site known as a taste receptor were used. Further, LexAop-TNT means that Drosophila inhibiting taste receptors by expressing TNT toxin in the presence of LexA protein was used.

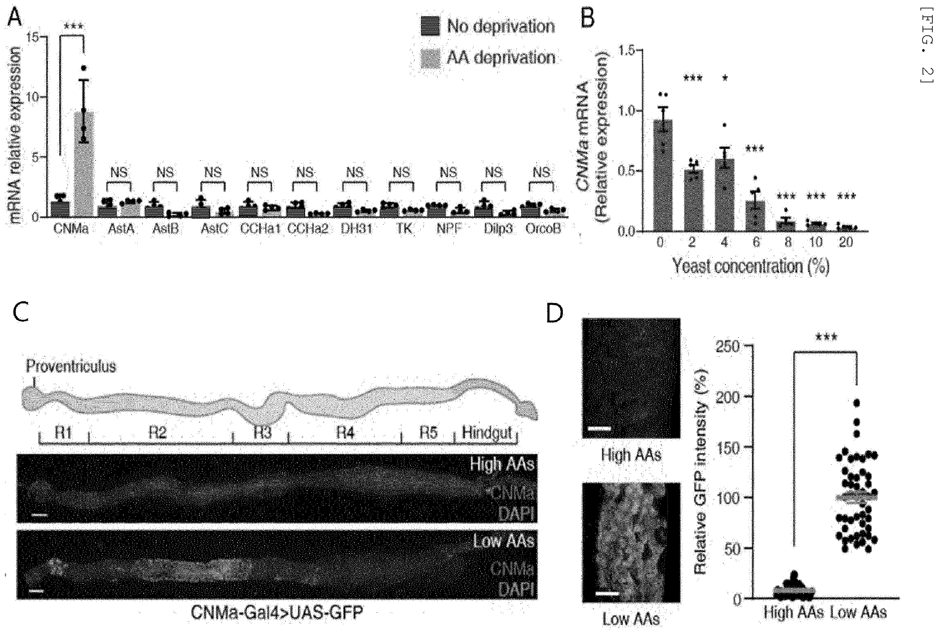

FIG. 2 shows results of excavation of CNMa hormone as an intestinal hormone induced by protein deficiency, and confirming the position of CNMa expression in the Drosophila gut. Specifically, FIG. 2A shows results of confirming mRNA expression levels of 11 intestinal hormones in respective conditions by feeding Drosophila with a protein-containing medium and a protein-deficient medium, and dissecting the Drosophila gut to investigate the expression levels of the 11 intestinal hormones through Real-Time qPCR. Only CNMa among the 11 intestinal hormones had an increased expression in the protein-deficient condition. FIG. 2B shows results of measuring the expression level of CNMa mRNA in the Drosophila gut under each medium condition after feeding Drosophila with the media having the same calorific value but different protein (yeast) content. As the protein content in the medium was increased, the mRNA expression of CNMa hormone was decreased. FIGS. 2C to D show results of confirming the expression level of the CNMa hormone expressed in the Drosophila gut under the conditions of high protein medium (High AAs) and low protein medium (Low AAs) using CNMa-Gal4 > UAS-GFP Drosophila line. In the Drosophila gut, the expression of CNMa was specifically increased in intestinal enterocytes of the R2 region.

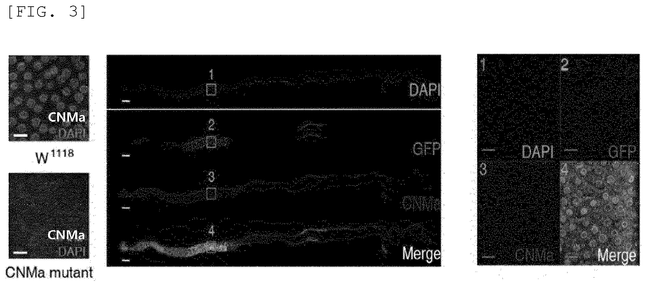

FIG. 3 shows results of confirming the expression of endogenous CNMa by preparing a CNMa-specific antibody, and results of reconfirming that the expression of CNMa hormone occurs in intestinal epithelial cells of the R2 region of the Drosophila gut as in the CNMa-Gal4 expression region.

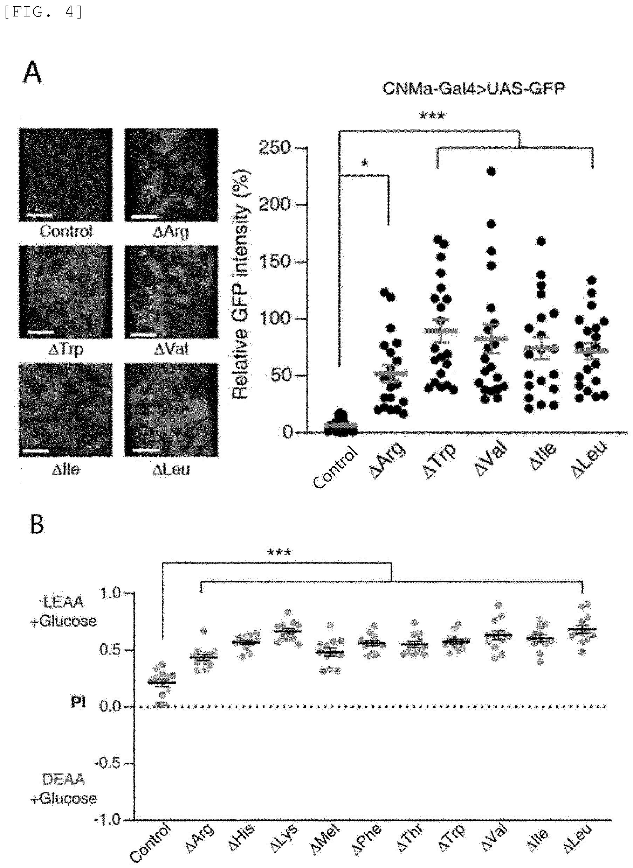

FIG. 4 shows an increase in the expression of CNMa hormone and a change in L-EAA preference index induced by the deficiency of a single essential amino acid. Specifically, FIG. 4A shows results of confirming a change in the expression of CNMa hormone in the Drosophila intestinal R2 region after feeding Drosophila with a medium in which arginine, tryptophan, valine, isoleucine and leucine are removed from Holidic media (Chemical defined media). It was confirmed that the expression of CNMa hormone was induced only by the deficiency of the single essential amino acid. FIG. 4B shows results of confirming that, even if only one type of essential amino acid is deficient, the L-EAA preference index of Drosophila is increased.

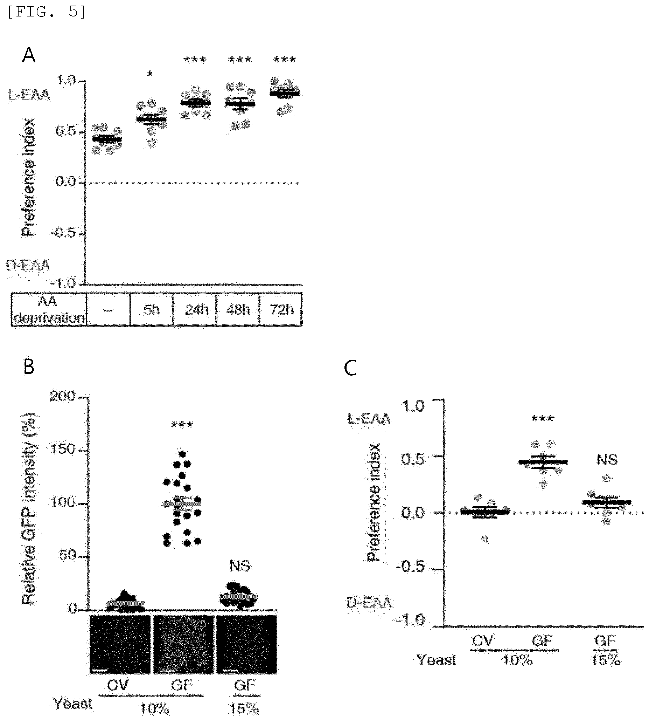

FIG. 5 shows results of comparison of the CNMa hormone expression levels and differences in the L-EAA preference index between normal Drosophila and sterile Drosophila. Specifically, FIG. 5A shows results of observing the L-EAA preference index after placing sterile Drosophila in a protein-containing medium and a protein-deficient medium by time. FIG. 5B shows results of confirming the expression level of CNMa in the gut after feeding normal Drosophila (CV) and sterile Drosophila (GF) with food having a 10% concentration of yeast. The expression of CNMa hormone in sterile Drosophila was increased even though the medium with the same protein content was fed, and this increased expression of CNMa was offset when yeast was additionally added to the medium. FIG. 5C shows results of confirming the L-EAA preference index in the intestine after feeding normal Drosophila (CV) and sterile Drosophila (GF) with food having a 10% concentration of yeast. The preference for L-EAA feeding of sterile Drosophila was increased even though the medium with the same protein content was fed, and this result was offset when the yeast was additionally added to the medium. These results suggest that enterobacteriaceae affect the feeding preference to satisfy the host's nutrition by offsetting the L-EAA deficiency.

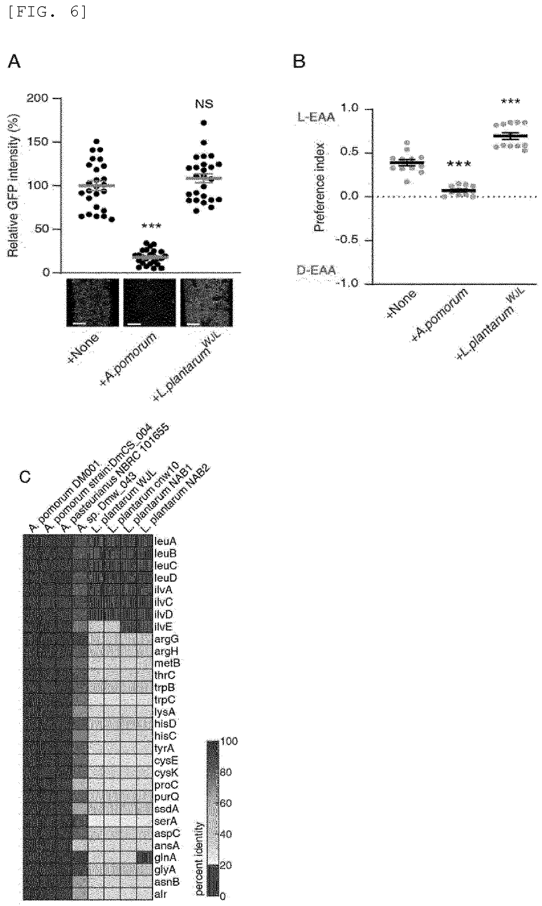

FIG. 6 shows analysis results of comparison of differences in the CNMa expression levels and L-EAA nutritional fulfillment behavior between Acetobacter-introduced Drosophila and Lactobacillus-introduced Drosophila, and comparison of amino acid biosynthesis pathways of *Acetobacteraceae* and *Lactobacillaceae.* Specifically, FIG. 6A shows the expression levels of intestinal CNMa hormone of sterile Drosophila (+None), Drosophila (+ *A.pomorum*) introduced with *A.pomorum,* and Drosophila (+*L.plantarum* WJL) introduced with *L.plantarum* WJL. FIG. 6B shows observation results of L-EAA nutritional fulfillment behavior of sterile Drosophila (+None), Drosophila (+*A.pomorum*) introduced with *A.pomorum,* and Drosophila (+*L.plantarum* WJL) introduced with *L.plantarum* WJL. It was confirmed that Acetobacter offsets L-EAA deficiency, but Lactobacillus did not offset the same, thus inducing the feeding preference to satisfy the host's nutrition. FIG. 6C shows analysis results of the genes involved in the amino acid biosynthesis pathway of *Acetobacteraceae, Lactobacillaceae* by comparative genomic anlaysis. Based on the gene of *A.pomorum DM001,* the higher the nucleotide sequence similarity of the gene, the higher the blue color, and when the similarity is less than 20%, it was indicated with red color. It was confirmed that *Lactobacillaceae* commonly lack genes related to branched-chain amino acid (BCAA) biosynthesis.

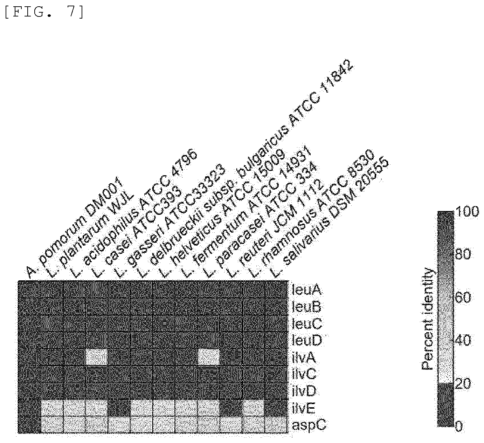

FIG. 7 shows results of comparative analysis of the BCAA biosynthetic pathway genes of *Lactobacillus* strains from the Food Notice. The genes involved in the amino acid biosynthesis pathway of 11 species of *Lactobacillus* were analyzed by comparative genomic analysis. Based on the gene of *A.pomorum DM001,* the higher the nucleotide sequence similarity of the gene, the higher the blue color, and when the similarity is less than 20%, it was indicated with the red color. It was confirmed that 11 *Lactobacillus* strains commonly lack genes related to branched-chain amino acid (BCAA) biosynthesis.

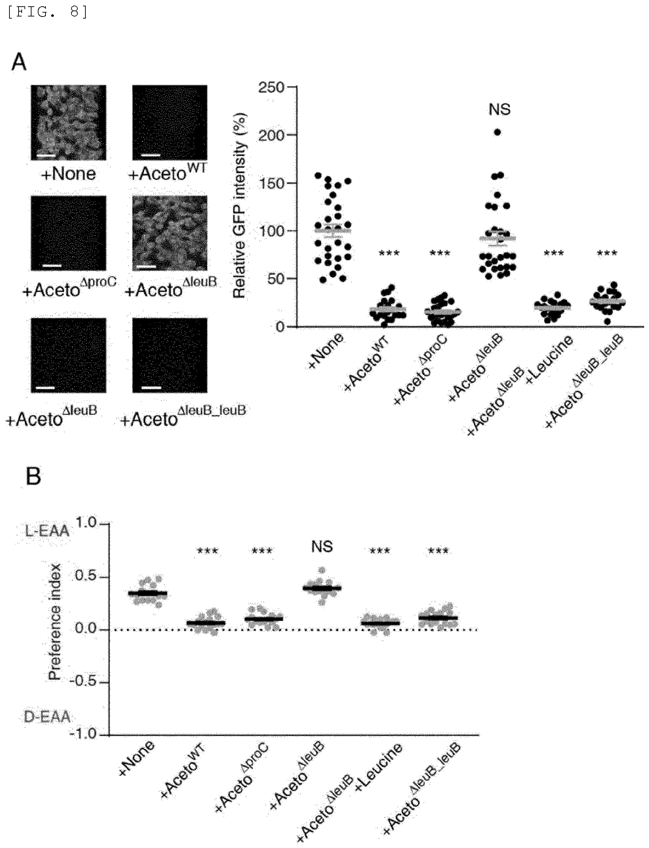

FIG. 8 shows results of confirming the CNMa hormone expression level and L-EAA nutritional fulfillment behavior of Drosophila introduced with an *Acetobacter* mutant strain that is regulated not to biosynthesize leucine as one type of BCAA. FIGS. 8A to 8B show: (A) CNMa hormone expression levels; and (B) L-EAA preference index of the sterile Drosophila (+None), Drosophila (+Aceto ^{WT}) introduced with control *Acetobacter,* Drosophila (+Aceto ^{ΔleuB}) introduced with Aceto ^{ΔleuB} that cannot biosynthesize leucine, and Drosophila (Aceto ^{ΔproC}) introduced with Aceto ^{ΔproC} that cannot biosynthesize proline, respectively. It could be confirmed that the CNMa expression and the L-EAA preference index of Aceto ^{ΔleuB}-introduced Drosophila were reduced when leucine was added to the medium or when bacteria genetically reintroduced with (+Aceto ^{ΔleuB+Leucine}) -deficient leuB were prepared and introduced into Drosophila (+Aceto ^{ΔleuB_leuB}).

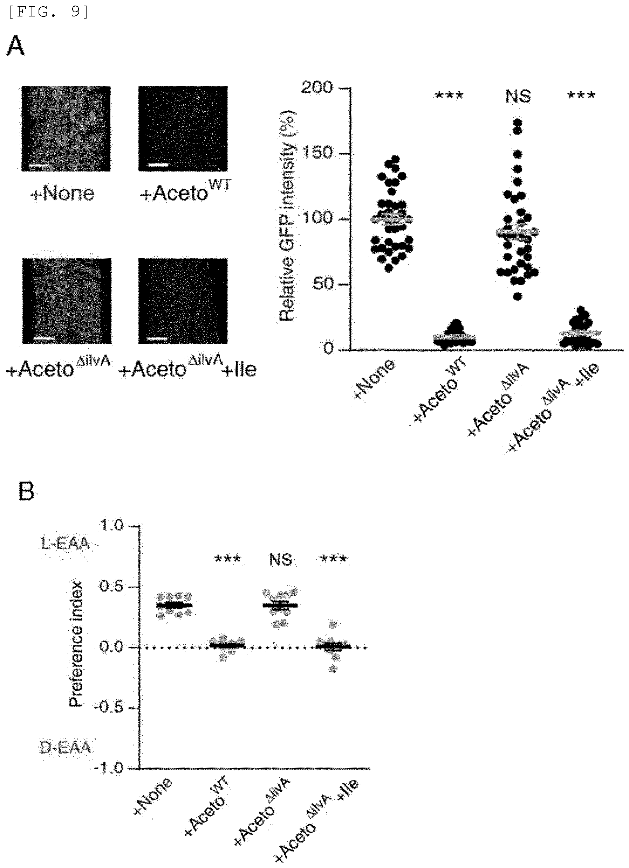

FIG. 9 shows results of confirming the CNMa hormone expression level and L-EAA nutritional fulfillment behavior of Drosophila introduced with an *Acetobacter* mutant strain that is regulated not to biosynthesize isoleucine as one type of BCAA. Specifically, FIGS. 9A to 9B show (A) the CNMa hormone expression levels and (B) L-EAA preference indexes of sterile Drosophila (+None), Drosophila (+Aceto ^{WT}) introduced with control Acetobacter, and Drosophila (+Aceto ^{ΔilvA}) introduced with Aceto ^{ΔilvA} that cannot biosynthesize isoleucine, respectively. The CNMa expression and L-EAA preference index of Drosophila introduced with Aceto ^{ΔilvA} were restored to the level of Drosophila introduced with Aceto ^{WT} when isoleucine was added to the medium (+Aceto ^{ΔleuB+Ile}).

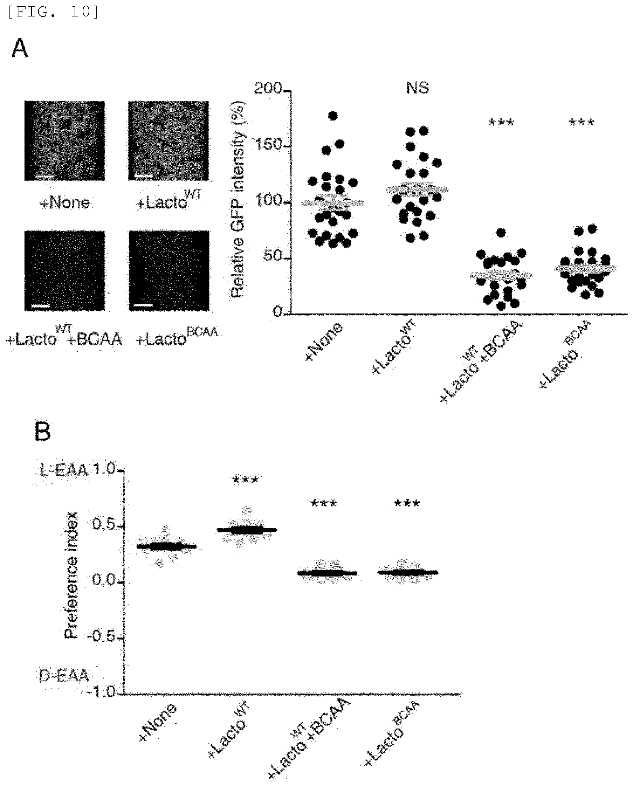

FIG. 10 shows changes in the CNMa hormone expression and L-EAA nutritional fulfillment behavior index according to BCAA biosynthesis ability of *Lactobacillus.* Specifically, FIGS. 10A to 10B show observation results of (A) CNMa hormone expression levels and (B) L-EAA nutritional fulfillment behavior of sterile Drosophila (+None), control *Lactobacillus-*introduced Drosophila (+Lacto ^{WT}), and in Drosophila (+Lacto ^{BCAA}) introduced with mutant *Lactobacillus* (Lacto ^{BCAA}) that acquired BCAA biosynthesis ability, respectively. It could be seen that Drosophila introduced with Lacto ^{BCAA}, which has acquired BCAA biosynthesis ability, offset L-EAA deficiency, thereby reducing the host's CNMa hormone expression and L-EAA nutritional fulfillment behavior index. Further, when BCAAs (isoleucine, leucine, valine) were added to the medium of Lacto ^{WT}-introduced Drosophila, CNMa expression and L-EAA nutritional fulfillment behavior index were reduced to those of Drosophila introduced with Lacto ^{BCAA}.

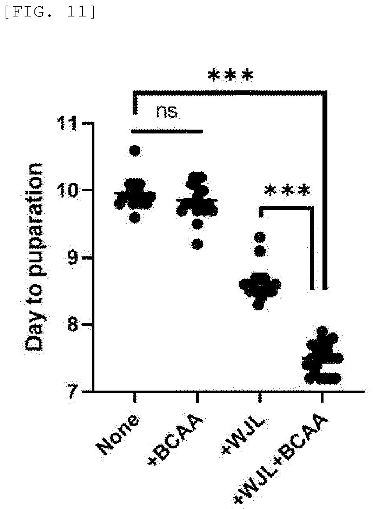

FIG. 11 shows results of confirming that the animal growth efficacy is enhanced when BCAA is added together with the lactic acid bacteria in the Drosophila model. When BCAA was added to sterile Drosophila EGG (+BCAA), when *L. plantarum* WJL was introduced alone (+WJL), or when bacteria and BCAA were given together (+WJL+BCAA), pupae formation was observed every 12 hours and the average time of occurrence was measured. It could be seen that, when BCAA as a nutrient is added (+BCAA), it did not help the growth of fruit flies at all. On the other hand, it could be confirmed that, when lactic acid bacteria and BCAA were given together (+WJL+BCAA), the growth of Drosophila was statistically and significantly improved.

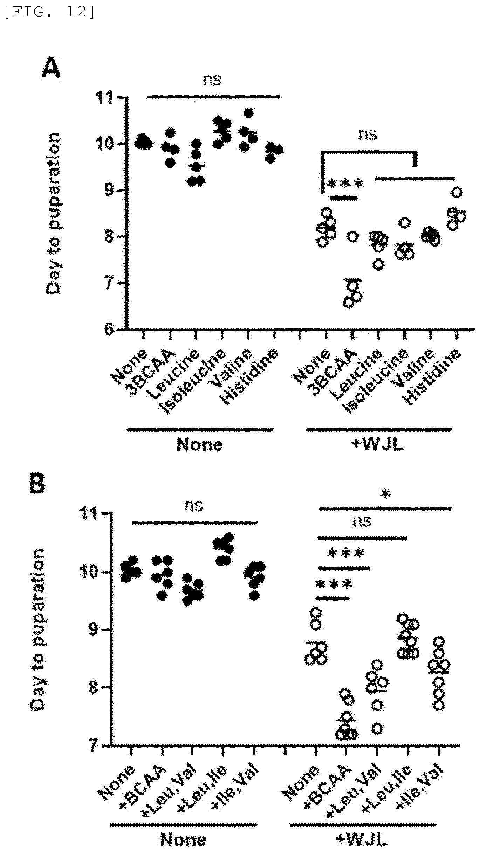

FIG. 12 shows results of confirming the growth promoting efficacy when at least two or more BCAAs among leucine, isoleucine and valine and the lactic acid bacteria are used in combination. Specifically, FIG. 12A shows the growth promoting efficacy when treated with only one type of amino acid or treated with one type of amino acid in addition to lactic acid bacteria. FIG. 12B shows the growth promoting efficacy when treated with two or more types of BCAAs without lactic acid bacteria, or treated with two or more types of BCAAs in addition to lactic acid bacteria.

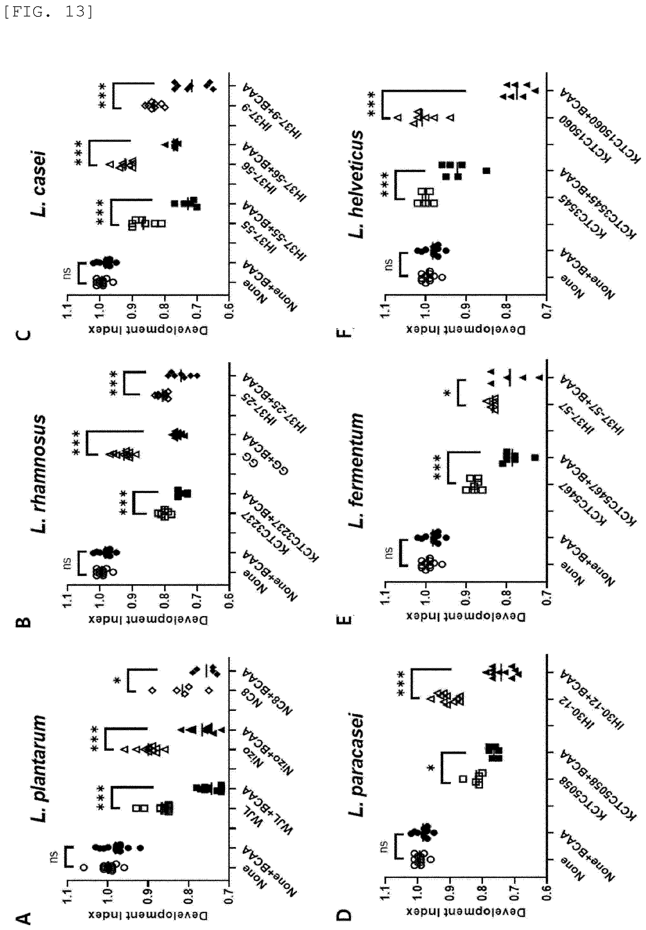

FIG. 13 shows results of confirming whether the growth promoting efficacy of lactic acid bacteria is increased when various types of lactic acid bacteria are added together with BCAA.

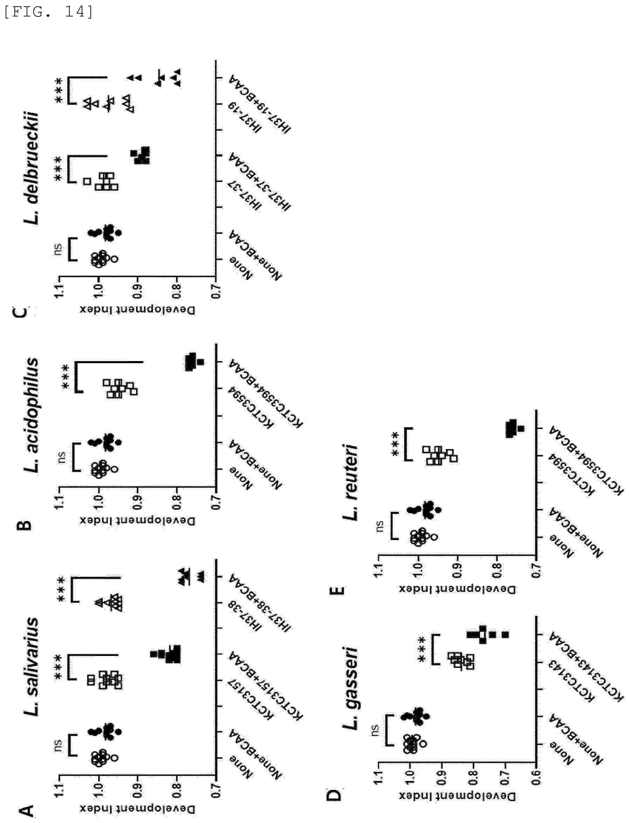

FIG. 14 shows results of confirming whether the growth promoting efficacy of lactic acid bacteria is increased when various types of lactic acid bacteria are added together with BCAA.

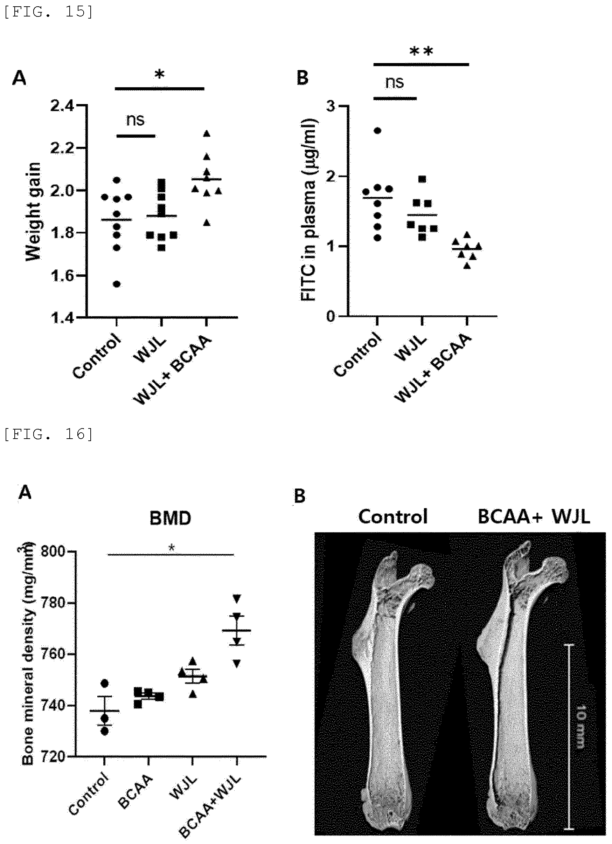

FIG. 15 shows results of confirming whether the growth promoting efficacy or leaky gut inhibitory efficacy of lactic acid bacteria is increased when BCAA is added together with the lactic acid bacteria in a mouse animal model. Specifically, FIG. 15A shows results of measuring a change in body weight of young mice after 12 weeks. FIG. 15B shows results of measuring excavation of fluorescein isothiocyanate-dextran (FITC) flowing into the blood through the intestine by feeding the mouse with FITC, wherein the higher the numerical value, the more severe the leaky gut. When lactic acid bacteria and BCAA were given together (+WJL+BCAA), it could be seen that the weight of the mouse was increased while decreasing the degree of leaky gut. The weight gain and the decrease in the leaky gut showed statistically significant results.
FIG. 16 shows results of measuring Femur cortical bone mineral density (BMD) when BCAA is added together with the lactic acid bacteria in a mouse animal model. When lactic acid bacteria and BCAA were treated together (+WJL+BCAA), it could be seen that the bone density of the mice was increased statistically significantly. Specifically, FIG. 16B shows a representative photograph of Femur.

### [Mode for Carrying out Invention]

Hereinafter, the present invention will be described in detail.

The present invention provides a composition for enhancing the efficacy of lactic acid bacteria that include at least two branched-chain amino acids (BCAAs) selected from the group consisting of leucine, isoleucine and valine.

The term "efficacy of lactic acid bacteria" refers to the function or effect of lactic acid bacteria that act on a specific subject or individual, and can also be expressed as "physiologically active function of lactic acid bacteria." The term "biological activity" refers to the property of a specific drug or substance acting on an organism.

The composition for enhancing the efficacy of lactic acid bacteria may be to enhance the growth promoting efficacy of lactic acid bacteria, or to improve the intestinal damage inhibitory efficacy of lactic acid bacteria.

The efficacy of lactic acid bacteria may be to promote the growth of a subject or inhibit intestinal damage. Specifically, the efficacy of lactic acid bacteria may be to increase at least one of the subject's body weight or bone density, or to inhibit leaky gut of the subject.

The efficacy of lactic acid bacteria may be to increase at least one of body weight and bone density of a nutrient-deficient subject, or to inhibit leaky gut of the nutrient-deficient subject.

The term "growth promoting efficacy of lactic acid bacteria" refers to the efficacy of lactic acid bacteria to promote the growth of other organisms or subjects. For example, the composition for enhancing the efficacy of lactic acid bacteria according to the present invention can increase the efficacy of lactic acid bacteria that promotes the growth of a subject for the lactic acid bacteria-treated subject when treated in combination with the lactic acid bacteria. Specifically, compared to the case where the lactic acid bacteria are administered alone to any subject, when the efficacy enhancing composition of the present invention is used in combination with the lactic acid bacteria to treat any subject, the growth promoting effect of the subject is remarkably excellent. According to one embodiment, it was confirmed that, when using the composition for enhancing the efficacy of lactic acid bacteria according to the present invention together with the lactic acid bacteria, a bone density is excellent and remarkably increased and weight gain effect appears in the subject treated with the above combination, compared to the case where only the lactic acid bacteria were treated. High bone density helps to increase height, and the bone density is used as an important index for growth by those skilled in the art.

The term "intestinal damage inhibitory efficacy of lactic acid bacteria" refers to the efficacy of lactic acid bacteria in which lactic acid bacteria inhibit intestinal damage of other organisms or subjects. For example, the composition for enhancing the efficacy of lactic acid bacteria of the present invention can increase the efficacy of lactic acid bacteria inhibiting intestinal damage of the subject in the subject treated with lactic acid bacteria when using in combination with the lactic acid bacteria. Specifically, compared to the case where the lactic acid bacteria are administered alone to any subject, when the efficacy enhancing composition of the present invention is used in combination with the lactic acid bacteria to treat any subject, the effect for inhibiting intestinal damage of the subject is remarkably excellent. Intestinal damage may be leaky gut and intestinal inflammation, but it is not limited thereto.

According to an embodiment, it was confirmed that, when using the composition for enhancing the efficacy of lactic acid bacteria according to the present invention together with the lactic acid bacteria, a significantly superior effect of inhibiting leaky gut appears in the subjects treated in combination with the lactic acid bacteria, compared to the case where only the lactic acid bacteria were treated.

The composition for enhancing the efficacy of lactic acid bacteria may serve as an adjuvant capable of further improving the effect of lactic acid bacteria (e.g., growth promoting effect, intestinal damage inhibitory effect).

The composition for enhancing the efficacy of lactic acid bacteria may be used in combination with the lactic acid bacteria, and may serve as an adjuvant capable of further improving the effect resulted from lactic acid bacteria alone by using in combination with the lactic acid bacteria.

The composition for enhancing the efficacy of lactic acid bacteria may be treated with lactic acid bacteria simultaneously, separately or sequentially.

The subject may be an animal including a human, for example, a human, a dog, a cat, a horse, a cow, a rabbit, a goat, but it is not limited thereto.

The subject may be nutritionally deficient or nutritionally unbalanced. For example, the subject may be an individual deficient in protein nutrition.

The composition for enhancing the efficacy of lactic acid bacteria is used in combination with the lactic acid bacteria in order to improve the efficacy of lactic acid bacteria to nutritionally deficient or nutritionally unbalanced organisms or subjects by increasing at least one of body weight or bone density of the nutrient-deficient subject, or by suppressing the leaky gut of the nutrient-deficient subject. "Nutrition deficiency" may refer to a condition in which one or more essential nutrients or calories are deficient, and may occur by, for example, insufficient intake of nutrients, malabsorption or processing disorders and the like. In the case of the nutrient-deficient subject, symptoms such as underweight, protrusion of bones, dry skin and decreased elasticity, dry hair, or the like may appear. "Nutritional unbalance" refers to an imbalance between nutrients required for a body and nutrients obtained from the body, and may include both overnutrition and undernutrition.

The composition for enhancing the efficacy of lactic acid bacteria may enhance the efficacy of lactic acid bacteria to promote the growth of nutrient-deficient or nutrient-unbalanced subjects or to enhance the efficacy of inhibiting intestinal damage.

The age of the subject is not limited, but the subject may include individuals such as a child, an adult excluding elderly, or the elderly.

The subject may be an individual of childhood. Childhood refers to a period of conception, birth and adulthood. The childhood may include cellular, prenatal, neonatal, infancy, childhood and puberty periods.

The composition for enhancing the efficacy of lactic acid bacteria may improve the activity of lactic acid bacteria *in vitro.* For example, the composition for enhancing the efficacy of lactic acid bacteria can promote the growth of cells or tissues treated with lactic acid bacteria *in vitro.* For example, the composition for enhancing the efficacy of lactic acid bacteria may inhibit damage to intestinal-related cells or tissues treated with lactic acid bacteria *in vitro.*

Lactic acid bacteria are bacteria that produce lactic acid using sugar as an energy source, and may be found in the digestive organs, oral cavity, etc. of humans or mammals and are widely distributed in nature such as diverse fermented food products. Lactic acid bacteria is one of the microorganisms that have been widely used for a long time by mankind, and has good function of preventing decay in the intestines while not generating substances harmful to the intestines of humans or animals. Lactic acid bacteria mean beneficial bacteria capable of producing lactic acid.

The lactic acid bacteria may be *Lactobacillaceae, Enterococcaceae, Streptococcaceae,* or *Bifidobacteriaceae.*

The lactic acid bacteria may be selected from the group consisting of *Lactobacillus spp., Lactococcus spp., Enterococcus spp., Streptococcus spp.,* and *Bifidobacterium spp.*

Lactobacillus genus may be selected from the group consisting of *Lactiplantibacillus plantarum, Lactobacillus acidophilus, Lactobacillus casei, Lactobacillus gasseri, Lactobacillus delbrueckii subsp. Bulgaricus, Lactobacillus helveticus, Lactobacillus fermentum, Lactobacillus paracasei, Lactobacillus reuteri, Lactobacillus rhamnosus* and *Lactobacillus salivarius,* but it is not limited thereto. According to one embodiment, the composition of the present invention may enhance the efficacy of lactic acid bacteria when it is treated in combination with at least one lactic acid bacteria selected from the group consisting of *Lactiplantibacillus plantarum, Lactobacillus paracasei, Lactobacillus rhamnosus, Lactobacillus fermentum, Lactobacillus casei, Lactobacillus acidophilus, Lactobacillus gasseri, Lactobacillus delbrueckii subsp. Bulgaricus, Lactobacillus reuteri, Lactobacillus helveticus,* and *Lactobacillus salivarius.*

The lactic acid bacteria may be isolated from plants, fermented foods, animals including mammals or arthropods, or may be present in the intestine of the subject. The composition for enhancing the efficacy of lactic acid bacteria according to the present invention may improve the efficacy of the isolated lactic acid bacteria, and may enhance the efficacy of the lactic acid bacteria present in the intestine of the subject.

The lactic acid bacteria may be live or dead bacteria, and the form thereof is not limited.

*L. plantarum* WJL used in one embodiment may be prepared by a known manufacturing method (Eun-Kyoung Kim et al., Genome Announcements, November/December 2013, Vol.1, no. 6 e00937-13, GenBank AUTE00000000, Lactobacillus plantarum WJL, whole genome shotgun sequencing project). *L. plantarum* WJL may be isolated from Drosophila. *L. plantarum* WJL may be a deposited strain (Accession No.: KCTC14442BP, Depositary Organization: Biological Resources Center, Deposit Date: January 11, 2021).

The present inventors consider that lactic acid bacteria lack branched-chain amino acid biosynthetic enzyme-related genes through genetic analysis of lactic acid bacteria and the lactic acid bacteria cannot synthesize branched-chain amino acids, and predict that the efficacy of lactic acid bacteria can be improved if the branched-chain amino acid is used in combination with the lactic acid bacteria, therefore, the branched-chain amino acid was selected as an active ingredient of the composition for enhancing the efficacy of lactic acid bacteria according to the present invention. The composition for enhancing the efficacy of lactic acid bacteria may exhibit the effect of improving the efficacy of lactic acid bacteria by using in combination with the amino acids not biosynthesized in lactic acid bacteria.

The term "branched-chain amino acid (BCAA)" is an amino acid having an aliphatic side chain with a branch that is a central carbon atom bonded to three or more carbon atoms. Protein-producing BCAAs include the essential amino acids such as leucine, isoleucine and valine, while non-protein BCAAs include 2-aminoisobutyric acid. The branched-chain amino acid may be a free amino acid. "Free amino acid" means an amino acid that is not a part of a protein.

The composition for enhancing the efficacy of lactic acid bacteria may include at least two branched-chain amino acids selected from the group consisting of leucine, isoleucine and valine.

Specifically, the composition for enhancing the efficacy of lactic acid bacteria may include: leucine and isoleucine; leucine and valine; isoleucine and valine; or leucine, isoleucine and valine.

When the composition for enhancing the efficacy of lactic acid bacteria according to the present application includes all of two or more of leucine, isoleucine and valine, the efficacy of lactic acid bacteria can be more effectively improved compared to the case where they are included alone.

A composition for enhancing the efficacy of lactic acid bacteria (e.g., a composition for improvement of the growth promoting efficacy of lactic acid bacteria or for improvement of the intestinal damage inhibitory efficacy of lactic acid bacteria) may include leucine, isoleucine and valine.

When the composition for enhancing the efficacy of lactic acid bacteria of the present application includes all of leucine, isoleucine and valine, the activity of lactic acid bacteria can be most effectively improved.

According to one embodiment, compared to the case where a composition containing leucine, isoleucine or valine alone is treated in combination with the lactic acid bacteria, when a composition containing two or more of leucine, isoleucine and valine is treated in combination with the lactic acid bacteria, it can be seen that the growth of the subject was significantly enhanced.

The branched-chain amino acids included in the composition of the present invention may serve to improve the function of lactic acid bacteria in the intestine, rather than simply being included for nutritional supplementation of the subject.

As described above, when the branched-chain amino acid lacking synthesis in lactic acid bacteria is used in combination with the lactic acid bacteria, the physiological activity of the lactic acid bacteria (e.g., growth promoting effect or intestinal damage inhibitory effect) is enhanced, and even if other proteins excluding the branched-chain amino acids are not used together, the composition of the present invention alone may sufficiently exhibit effects of enhancing the efficacy of lactic acid bacteria. Accordingly, the composition for enhancing the efficacy of lactic acid bacteria according to the present application may exhibit excellent effects of enhancing the efficacy of lactic acid bacteria, and has an economical advantage in terms of consumption of raw materials.

For example, the composition for enhancing the efficacy of lactic acid bacteria may not include other proteins (e.g., whey protein) in addition to the branched-chain amino acids. The composition for enhancing the efficacy of lactic acid bacteria can significantly enhance the efficacy of lactic acid bacteria even if it does not include other proteins (e.g., whey protein) in addition to the same. In this case, costs of raw materials for other proteins can be reduced, thereby resulting in excellent economic advantages. Further, if other proteins are included, it is possible to reduce deterioration in functions of branched-chain amino acids, or the possibility of unexpected side effects.

"Whey protein" is a milk protein from which casein has been removed. 80% of whey protein consists of lactalbumin and lactoglobulin, and further includes other components such as proteose and peptone which are not coagulated by acid or heat. Although whey protein has advantages such as improvement of recovery speed after exercise, satiety and weight control, etc., whereas lactose intolerant subjects lacking lactose degrading enzymes or subjects having milk allergy may experience symptoms such as diarrhea or abdominal pain when whey protein is taken, thus should be careful. Further, subjects with kidney disease or liver disease who require protein limitation also need to be careful when whey protein is taken.

The composition for enhancing the efficacy of lactic acid bacteria according to the present invention may not contain whey protein, such that problems possibly occurring when whey protein is included (diarrhea, abdominal pain, allergy, etc.) are not caused, and the growth of lactic acid bacteria may be promoted or intestinal damage inhibitory efficacy can be improved.

For another example, the composition for enhancing the efficacy of lactic acid bacteria may be composed of at least two branched-chain amino acids selected from the group consisting of leucine, isoleucine and valine.

Further, the present invention provides a method for enhancing the efficacy of lactic acid bacteria, which includes; administering the composition for enhancing the efficacy of lactic acid bacteria described above to a subject.

Since the "composition for enhancing the efficacy of lactic acid bacteria," the "lactic acid bacteria," and the "efficacy of lactic acid bacteria" have been described above, these will not be described in detail.

The lactic acid bacteria may be present in the intestine of the subject.

The subject may be an animal including a human, for example, a human, a dog, a cat, a horse, a cow, a rabbit, a goat, but it is not limited thereto.

The subject may be an individual with growth retardation or occurrence of intestinal damage.

The subject may be an individual who has inhibited growth or an intestinal damage occurring due to nutritional deficiency or nutritional unbalance.

The method for enhancing the efficacy of lactic acid bacteria may not include administering a polypeptide or protein (e.g., whey protein) other than the branched-chain amino acids and lactic acid bacteria.

Further, the present invention provides a pharmaceutical composition for promoting growth or inhibiting intestinal damage, which includes the above-described composition for enhancing the efficacy of lactic acid bacteria; and lactic acid bacteria.

The pharmaceutical composition of the present application may promote the growth of a subject to which the pharmaceutical composition is administered, or inhibit intestinal damage by including both the composition for enhancing the efficacy of lactic acid bacteria that improves the growth promoting efficacy of the lactic acid bacteria and the intestinal damage inhibitory efficacy, and the lactic acid bacteria.

Since the "composition for enhancing the efficacy of lactic acid bacteria" and "lactic acid bacteria" have been described above, these will not be described in detail.

The growth promoting effect may refer to an effect of promoting the growth of a subject including a human, to which the pharmaceutical composition is administered.

The growth promotion may be at least one of weight gain, height increase, and bone density increase.

According to one embodiment, it was confirmed that the body weight and bone density increase in animals using the composition for enhancing the efficacy of lactic acid bacteria of the present application and lactic acid bacteria in combination.

The intestinal damage inhibitory effect may refer to an effect of inhibiting intestinal damage of a subject including a human, to which the pharmaceutical composition is administered.

Intestinal damage may be inflammatory bowel disease, environmental enteropathy, or leaky gut syndrome, but it is not limited thereto.

According to one embodiment, it was confirmed that leaky gut was inhibited in animals that received administration of the composition for enhancing the efficacy of lactic acid bacteria according to the present application in combination with the lactic acid bacteria.

Further, the present invention provides a composition for prevention or treatment of at least one disease selected from the group consisting of growth disorders, hypogrowth, osteoporosis, osteomalacia, osteopenia, environmental enteropathy and leaky gut syndrome, which includes: the above-described composition for enhancing the efficacy of lactic acid bacteria; and lactic acid bacteria. The above function may include effects expressed by improving physiologically active effects (e.g., growth promoting effect and intestinal damage inhibitory effect) of lactic acid bacteria in the subject to which the pharmaceutical composition is administered.

The term "prevention" refers to any action that inhibits disease or delays its progression by administration of the composition for enhancing the efficacy of lactic acid bacteria and the lactic acid bacteria. The term "treatment" refers to any action in which symptoms for a disease are improved or beneficially changed by administration of the composition for enhancing the efficacy of lactic acid bacteria and the lactic acid bacteria.

The disease may be caused by nutritional deficiency or nutritional unbalance. Since "nutrition deficiency or nutritional unbalance" has been described above, this will not be described in detail.

The disease may occur in animals, including humans, and the age thereof is not limited.

The disease may occur in children, adults excluding the elderly, elderly individuals. Specifically, this may include diseases occurring in an individual of childhood. Since the term "childhood" has been described above, this will not be described in detail.

The pharmaceutical composition of the present application may be administered to a nutrient-deficient or nutrient-unbalanced subject.

The pharmaceutical composition of the present application may be administered to an individual of childhood.

The pharmaceutical composition of the present application may be for the prevention or treatment of at least one disease selected from the group consisting of growth disorders, hypogrowth, osteoporosis, osteomalacia, osteopenia, environmental enteropathy and leaky gut syndrome in the individual of childhood.

Leaky gut syndrome is a disease occurring when a space is created between cells in the membrane that covers the intestinal lining. This is a disease in which the function of the intestine is deteriorated while increasing the intestinal permeability, which in turn causes disturbances in the absorption of water and nutrients and thus the immune system.

The environmental enteropathy (EE) is a disease that causes low growth and intellectual decline in children due to malnutrition and intestinal infection at the same time, and it is difficult to improve the environmental enteropathy only by simply supplying nutrients to the subjects.

The branched-chain amino acid contained in the pharmaceutical composition of the present invention may exhibit excellent effects in improving the environmental enteropathy by improving the efficacy of lactic acid bacteria, not simply for the purpose of supplying nutrients.

According to one embodiment, the pharmaceutical composition of the present application may include: at least two branched-chain amino acids selected from the group consisting of leucine, isoleucine and valine; and at least one lactic acid bacteria selected from the group consisting of *Lactiplantibacillus plantarum, Lactobacillus paracasei, Lactobacillus rhamnosus, Lactobacillus fermentum, Lactobacillus casei, Lactobacillus acidophilus, Lactobacillus gasseri, Lactobacillus delbrueckii subsp. Bulgaricus, Lactobacillus reuteri, Lactobacillus helveticus,* and *Lactobacillus salivarius.*

According to one embodiment, the pharmaceutical composition including: at least two branched-chain amino acids selected from the group consisting of leucine, isoleucine and valine; and at least one lactic acid bacteria selected from the group consisting of *Lactiplantibacillus plantarum, Lactobacillus paracasei, Lactobacillus rhamnosus, Lactobacillus fermentum, Lactobacillus casei, Lactobacillus acidophilus, Lactobacillus gasseri, Lactobacillus delbrueckii subsp. Bulgaricus, Lactobacillus reuteri, Lactobacillus helveticus,* and *Lactobacillus salivarius* may increase the development or body weight and bone density of a subject to which the composition is administered. According to one embodiment, the pharmaceutical composition including: at least two branched-chain amino acids selected from the group consisting of leucine, isoleucine and valine; and at least one lactic acid bacteria selected from the group consisting of *Lactiplantibacillus plantarum, Lactobacillus paracasei, Lactobacillus rhamnosus, Lactobacillus fermentum, Lactobacillus casei, Lactobacillus acidophilus, Lactobacillus gasseri, Lactobacillus delbrueckii subsp. Bulgaricus, Lactobacillus reuteri, Lactobacillus helveticus,* and *Lactobacillus salivarius* may inhibit leaky gut of the subject to which the composition is administered.

The pharmaceutical composition of the present invention may not contain other proteins (e.g., whey protein) in addition to the branched-chain amino acids and lactic acid bacteria. Even the composition does not contain the other proteins (e.g., whey protein), the branched-chain amino acid and lactic acid bacteria may significantly promote the growth of the subject or significantly inhibit intestinal damage, to which the composition is administered. In this case, costs of other proteins (e.g., whey protein) can be reduced, thereby resulting in excellent economic advantages.

The pharmaceutical composition of the present invention may further include pharmaceutically acceptable carriers, excipients or diluents. The pharmaceutically acceptable carrier may be those commonly used in formulation, for example, saline, sterile water, Ringer's solution, buffered saline, cyclodextrin, dextrose solution, maltodextrin solution, glycerol, ethanol, liposomes, and antioxidants, but it is not limited thereto.

The pharmaceutical composition of the present invention may be formulated as an injectable formulation, pill, capsule, granule or tablet, but it is not limited thereto.

The pharmaceutical composition of the present invention may be administered orally or parenterally (e.g., intravenously, subcutaneously, intraperitoneally or topically) according to a desired method, and may be appropriately selected by those skilled in the art.

The pharmaceutical composition of the present invention may be administered in a pharmaceutically effective amount.

The pharmaceutical composition of the present invention may be administered as an individual therapeutic agent or in combination with other therapeutic agents, may be administered sequentially or simultaneously with conventional therapeutic agents, and may be administered in single or multiple doses.

The effective amount of the pharmaceutical composition according to the present invention may vary depending on an age, sex, condition and/or body weight of the patient, absorption of the active ingredient in the body, inactivation rate and excretion rate, type of disease, and the drug to be used in combination. Typically, 0.001 to 150 mg, preferably 0.01 to 100 mg per 1 kg of body weight may be administered daily or every other day, or may be divided into 1 to 3 times a day. However, the dosage may be increased or decreased depending on the route of administration, severity of obesity, sex, body weight, age, etc., therefore, would not limit the scope of the present invention in any way.

Further, the present invention provides a method for promoting growth or inhibiting intestinal damage, which includes administering the composition for enhancing the efficacy of lactic acid bacteria and the lactic acid bacteria to a subject.

Since the "composition for enhancing the efficacy of lactic acid bacteria," "lactic acid bacteria," "promoting growth" and "inhibiting intestinal damage" have been described above, these will not be described in detail.

The subject may be an animal including a human, for example, a human, a dog, a cat, a horse, a cow, a rabbit, a goat, but it is not limited thereto.

The subject may be an individual with growth retardation or occurrence of intestinal damage.

The subject may be an individual who has inhibited growth or occurrence of an intestinal damage due to nutritional deficiency or nutritional unbalance.

The composition for enhancing the efficacy of lactic acid bacteria and the lactic acid bacteria may be administered simultaneously, separately or sequentially.

The method for promoting growth or inhibiting intestinal damage may not include administering a polypeptide or protein (e.g., whey protein) other than the branched-chain amino acids and lactic acid bacteria.

Further, the present invention provides a food composition for promoting growth or inhibiting intestinal damage, which includes: the above-described composition for enhancing the efficacy of lactic acid bacteria; and lactic acid bacteria.

The food composition of the present application may include both of the composition for enhancing the efficacy of lactic acid bacteria, which improves efficacy of promoting the growth of lactic acid bacteria and intestinal damage inhibitory efficacy, and the lactic acid bacteria, so as to promote the growth of a subject, to which the food composition is administered, or to inhibit intestinal damage.

Since the "composition for enhancing the efficacy of lactic acid bacteria," "lactic acid bacteria," "promoting growth," "inhibition of intestinal damage," and "intestinal damage" have been described above, these will not be described in detail.

The food composition of the present application may be for preventing or improving at least one disease selected from the group consisting of growth disorders, hypogrowth, osteoporosis, osteomalacia, osteopenia, environmental enteropathy and leaky gut syndrome. The above function may include effects expressed by enhancing the efficacy (e.g., growth promoting efficacy and intestinal damage inhibitory efficacy) of lactic acid bacteria in a subject to which the food composition is administered.

The term "improvement" refers to any action that reduces a parameter associated with a condition to be treated, for example, the symptom severity of a disease by intake of the food composition.

Since the "disease" has been described above, this will not be described in detail.

The food composition of the present application may be administered to an individual who is nutritionally deficient or nutritionally unbalanced.

The food composition of the present application may be administered to an individual of childhood.

The food composition of the present application may be for the prevention or improvement of at least one disease selected from the group consisting of growth disorders, hypogrowth, osteoporosis, osteomalacia, osteopenia, environmental enteropathy and leaky gut syndrome in the individual of childhood.

According to one embodiment, the food composition of the present application may include: at least two branched-chain amino acids selected from the group consisting of leucine, isoleucine and valine; and at least one lactic acid bacteria selected from the group consisting of *Lactiplantibacillus plantarum, Lactobacillus paracasei, Lactobacillus rhamnosus, Lactobacillus fermentum, Lactobacillus casei, Lactobacillus acidophilus, Lactobacillus gasseri, Lactobacillus delbrueckii subsp. Bulgaricus, Lactobacillus reuteri, Lactobacillus helveticus,* and *Lactobacillus salivarius.*

The food composition of the present invention may not contain other proteins (e.g., whey protein) in addition to the branched-chain amino acids and lactic acid bacteria, and even if the composition does not contain the other proteins (e.g., whey protein), it can significantly promote the growth of a subject to which branched-chain amino acids and lactic acid bacteria are administered, or significantly inhibit intestinal damage thereof. In this case, costs of other proteins (e.g., whey protein) can be reduced, thereby resulting in excellent economic advantages.

The food composition may be produced without limitation in various types of formulations as long as the formulations are recognized as food. For example, food may include meat, sausage, bread, chocolate, candy, snacks, confectionery, pizza, ramen, other noodles, gum, dairy products including ice cream, various soups, beverages, tea, drinks, alcoholic beverages, vitamin complexes, health functional foods, health foods, etc., and includes all foods in a conventional sense.

The food composition may be produced by a method commonly used in the related art.

The food composition may further include raw materials and ingredients commonly added in the related art. For example, the food composition may further include vitamins A, C, D, E, B1, B2, B6, B12, niacin, biotin, folate, and pantothenic acid. For example, the food composition may further contain minerals such as zinc (Zn), iron (Fe), calcium (Ca), chromium (Cr), magnesium (Mg), manganese (Mn), copper (Cu), chromium (Cr) and the like.

Hereinafter, the configuration and effect of the present invention will be described in more detail by way of examples. However, the following examples are provided for illustrative purposes only to help understanding of the present invention, and the scope and range of the present invention are not limited thereto.

### Example

The present inventors derived a combination of lactic acid bacteria and branched-chain amino acids that enhance the efficacy of lactic acid bacteria, as active ingredients to satisfy the nutrition of nutrient-deficient subjects through the following experiments. Prior to results of confirming whether the branched-chain amino acids promoting the efficacy of lactic acid bacteria and lactic acid bacteria exhibit efficacy enhancing effects of lactic acid bacteria, a process of selecting the lactic acid bacteria as a specific index appearing in the nutrient-deficient subject and also as one component satisfying the nutrition of the nutrient-deficient subject is firstly described.

In order to confirm whether the efficacy enhancing effects of the lactic acid bacteria are statistically significant by, comparing the control group and the experimental group, one-way ANOVA (with Dunnett's multiple comparison post-test) statistical analysis was performed. p value: *** (p < 0.001), **(P < 0.01), * (P < 0.05) showed statistically significant results. On the other hand, the experimental group not showing statistically significant results due to high p value was marked with "ns."

### 1. Identification of specific markers in nutrient-deficient subjects

### 1-1. L-EAA Preference in Nutrient-deficient subjects

Based on the feeding behavior of fruit flies (Drosophila) that specifically consume essential amino acids in a situation where there is a lack of protein in the body, the behavior index of nutrient-deficient subjects was observed. Specifically, Drosophila was placed in a protein-containing medium and a protein-deficient medium (a medium containing only glucose), and it was investigated which one among L-form essential amino acid (a form of amino acid that exists in nature, that is, L-form essential amino acid, L-EAA) and D-form essential amino acid (enantiomer of L-form essential amino acid, that is, D-EAA) is preferred as the food.

As a result, it was confirmed that the longer the subject exposed to the protein-deficient medium, the higher the preference index for L-EAA (see FIGS. 1A and 1B). Further, it was confirmed that the nutrient-deficient subject has preferred the intake of L-form essential amino acid among L-form amino acids. Further, it was confirmed that feeding preference to L-form non-essential amino acid (L-form Nonessential amino acid, L-NEAA) is not increased even in a protein deficiency situation (see FIGS. 1C to 1D). Further, as a result of comparing the L-EAA preference indexes of Drosophila subjects fed with the same calorie but different protein ratio, it could be confirmed that the preference index of L-EAA was decreased in the subjects who ingested high-protein content food. Through this, it was confirmed that the intake behavior of Drosophila supplementing L-EAA to satisfy protein nutrition is precisely regulated (see FIG. 1E). Further, L-EAA preference index was confirmed after inhibiting the activity of the existing Ir76b cells known as taste receptors that recognize proteins. Specifically, Drosophila in which Ir76b cells were inactivated by inducing tetanus toxin (TNT) overexpression, and the control Drosophila were placed in a protein-deficient situation to confirm L-EAA preference index. As a result, Drosophila in which Ir76b cells were inactivated showed a similar level of L-EAA preference index to the control Drosophila (see FIG. 1F). Through this, it was found that there would be other signaling pathways in addition to the previously known signaling pathways for recognizing nutritional deficiencies in the body.

### 1-2. CNMa hormone overexpression in nutrient-deficient subjects

In order to identify a new signaling pathway for recognizing nutritional deficiencies in the body, attention was paid to the gut as an endocrine organ where food is digested and absorbed, and various hormones are secreted. The protein-containing medium and the protein-deficient medium were fed to Drosophila, and the guts were dissected. Then, mRNA expression levels of 11 intestinal hormones under each condition were investigated through Real-Time qPCR. Primers used for Real-Time qPCR are listed in Table 1 below.

**[TABLE 1]**

| Hormone | Division of primers | Sequences | SEQ ID NO |
|---|---|---|---|
| CNMa | Forward primer | TGTGATCCTTGAGGGGATGT | SEQ ID NO: 1 |
| | Reverse primer | CAACAGCAGGAACAGAAGCA | SEQ ID NO: 2 |
| AstA | Forward | GAGGTCTCGTCCCTATCCTTC | SEQ ID NO: 3 |
| | primer | | |
| | Reverse primer | GATCTCGTTGTCCTGGTCGT | SEQ ID NO: 4 |
| AstB | Forward primer | TTTGGTGGCCAGTGGTAGTG | SEQ ID NO: 5 |
| | Reverse primer | GTCGCTCTCGCTAAGTTGGT | SEQ ID NO: 6 |
| AstC | Forward primer | GCGCCTATTTGAGGAGTCC | SEQ ID NO: 7 |
| | Reverse primer | CTTGACGTTTGCTCTCGGG | SEQ ID NO: 8 |
| CCHa1 | Forward primer | TCGTCGAGAATCGGAGGAT | SEQ ID NO: 9 |
| | Reverse primer | ACTGGCGCAATTGATA | SEQ ID NO: 10 |
| CCHa2 | Forward primer | CCCGTCAGGTGCTTTACAAAA | SEQ ID NO: 11 |
| | Reverse primer | GCGAGGTCGGTTAAACCATG | SEQ ID NO: 12 |
| DH31 | Forward primer | CGAAACATCGCATGGGTCTG | SEQ ID NO:13 |
| | Reverse primer | TCGGTCTCGGATCGTCGC | SEQ ID NO: 14 |
| TK | Forward primer | TACAAGCGTGCAGCTCTCTC | SEQ ID NO: 15 |
| | Reverse primer | CTCCAGATCGCTCTTCTTGC | SEQ ID NO: 16 |
| NPF | Forward primer | CATAAATCTAGGAAAAAATGCCAACA | SEQ ID NO: 17 |
| | Reverse primer | AAAACCGCGAGCAAATTCT | SEQ ID NO: 18 |
| Dilp3 | Forward primer | GTCCAGGCCACCATGAAGTTGTGC | SEQ ID NO: 19 |
| | Reverse primer | CTTTCCAGCAGGGAACGGTCTTCG | SEQ ID NO: 20 |
| OrcoB | Forward primer | TCCAAAAACAAGAGCCCCGA | SEQ ID NO: 21 |
| | Reverse primer | GTCTACTCTGCGTCCGCTTT | SEQ ID NO: 22 |

As a result, the expression level of CNMa among the 11 intestinal hormones in Table 1 was increased in the subjects under the nutritional deficient condition. Through this, it could be confirmed that the protein deficiency induced the CNMa hormone (see FIG. 2A). Further, as a result of providing Drosophila with media having the same calorific value but different protein (yeast) contents, and measuring the expression level of CNMa mRNA in the Drosophila gut under each medium condition, it was confirmed that the mRNA expression of CNMa hormone was decreased as the protein content in the medium was increased (see FIG. 2B). In order to investigate the expression level of the CNMa hormone *in vivo,* a Gal4 line (CNMa-Gal4) Drosophila whose expression is regulated by the CNMa promoter was prepared. Specifically, CNMa-gal4 was produced by replacing Mimic with gal4 using the recombinase-mediated cassette exchange (RMCE) system in CNMa [MI10321] (BDSC #54529) Drosophila in which Mimic is inserted into the first intron of the CNMa gene. As a result of investigating the expression of CNMa in the prepared Drosophila line through GFP, it could be confirmed that when the amount of protein taken by Drosophila in the actual Drosophila gut was small, the expression of the CNMa hormone remarkably was increased; on the other hand, when a high-protein diet was ingested, the expression of the CNMa hormone was sharply decreased (see FIGS. 2C and 2D). In particular, it was confirmed that the expression of CNMa was specifically increased in the intestinal epithelial cells (enterocytes) in the R2 region of the Drosophila gut (see FIGS. 2C and 2D). Further, in order to investigate the expression of endogenous CNMa, a CNMa-specific antibody was prepared and immunohistochemistry was performed. As a result, it was reconfirmed that the expression of the CNMa hormone occurred in the intestinal epithelial cells in the R2 region of the Drosophila gut, similar to the expression of the CNMa-Gal4 line (see FIG. 3). Through the above experimental results, it was confirmed that the deficiency of essential amino acids in the body can be recognized through the expression level of the CNMa hormone in the subject.

In order to determine whether the deficiency of a specific type of essential amino acid induces the expression of CNMa hormone, a medium deficient in arginine, tryptophan, valine, isoleucine or leucine alone was provided to Drosophila. As a result, it was confirmed that the expression of the CNMa hormone was induced in intestinal epithelial cells only by the deficiency of any single essential amino acid, not the deficiency of a specific essential amino acid (see FIG. 4A). Similarly, it was confirmed that only the deficiency of a single essential amino acid induced an increase in the preference index for L-EAA in Drosophila (see FIG. 4B). This demonstrates that the deficiency of the single essential amino acid alone indicates that Drosophila is malnourished.

### 2. Determine the effect of intestinal microbes on subject

The following experiment was performed to determine the effect of intestinal microbes on nutritional fulfillment in a subject.

Sterile Drosophila were prepared by collecting eggs laid by conventionally reared fly (CV) and rinsing the same in 3% NaClO solution and 70% ethanol solution alternately, in order to remove bacteria contained in the eggs and induce the development of flies in a sterile medium. It was confirmed how the prepared germ-free fly (GF) showed the preference for L-EAA in a protein-deficient medium and a protein-containing medium. As a result, sterile Drosophila showed increased basal L-EAA preference compared to the conventionally reared fly (CV) even under protein-rich conditions, and the preference index increased with increasing time. That is, it was confirmed that the sterile Drosophila always felt nutritional deficiency (see FIG. 5A). In the case of sterile Drosophila, it was found that intestinal bacteria play an important role in offsetting L-EAA deficiency, as they showed the feeding behavior with the preference for essential amino acids even in the absence of protein deficiency.

Further, the expression level of CNMa in the intestine was investigated after providing food with a 10% concentration of yeast to conventionally reared fly and sterile Drosophila. As a result, it was confirmed that the expression of CNMa hormone in sterile Drosophila was increased even though the medium with the same protein content was taken. When yeast was additionally added to the medium (Yeast 15%), it was confirmed that the increased expression of CNMa was decreased (see FIG. 5B). It was also confirmed that the L-EAA preference index of sterile Drosophila compared to the conventionally reared fly was increased even though the medium with the same protein content was taken. Further, when yeast was additionally added to the medium (Yeast 15%), L-EAA preference index was decreased in sterile Drosophila (see FIG. 5C). Through the above results, it was confirmed that the intestinal microbes offset L-EAA deficiency and affect the expression of CNMa hormone in Drosophila and the behavior to satisfy L-EAA nutrition.

### 3. Confirmation of nutritional fulfillment effect according to the types of introduced intestinal microbes

As the most representative examples of intestinal microbes, *Acetobacter pomorum* (*A.pomorum*) (Science, 2008 Feb 8;319(5864):777-82. doi: 10.1126/science.1149357. Epub 2008 Jan 24; Appl Environ Microbiol. 2008 Oct; 74(20): 6171-6177. Published online 2008 Aug 22. doi: 10.1128/AEM.00301-08), and *Lactobacillus plantarum* (*L. plantarum* WJL) (*KCTC14442BP*) were introduced into sterile Drosophila, respectively, thereby producing mono-association Drosophila. And then, the expression of CNMa hormone in the intestine was confirmed in each subject. As a result, it was confirmed that the Drosophila introduced with *A.pomorum* had a low level of CNMa hormone expression, but the Drosophila introduced with *L. plantarum* WJL had the same level of expression of the CNMa hormone as that of the sterile Drosophila (see FIG. 6A). *L. plantarum* WJL described in the present disclosure may be prepared by a known manufacturing method (Eun-Kyoung Kim et al., Genome Announcements, November/December 2013, Vol.1, no. 6 e00937-13, GenBank AUTE00000000, Lactobacillus plantarum WJL, whole genome shotgun sequencing project). *L. plantarum* WJL may be isolated from Drosophila.

Further, as a result of investigating the L-EAA nutritional fulfillment behavior index in each subject, it was confirmed that only Drosophila introduced with *A.pomorum* reduced the L-EAA nutritional fulfillment behavior index (see FIG. 6B). Further, when *L. plantarum* WJL was introduced alone, the preference index was higher than that of sterile Drosophila, which means that *L. plantarum* WJL can exacerbate the deficiency of essential amino acids in the intestine of the host animal. This corresponds to the expression pattern of the CNMa hormone.

According to the results of the high expression level of CNMa hormone and the high L-EAA preference index in the subjects introduced with *Lactobacillus plantarum* as an intestinal microorganism, the researchers found that, even if *Lactobacillus plantarum* alone was added to nutrient-deficient subjects, the nutritional fulfillment effect could have been expected not to appear. Accordingly, in order to determine the cause of the different levels of *CNMa* expression and L-EAA preference index between Drosophila introduced with *A.pomorum* and Drosophila introduced with *L. plantarum* WJL, genes of Lactobacillus and similar lactic acid bacteria were analyzed.

### 4. Genetic analysis of intestinal microbes and the difference in effect according to genetic traits of intestinal microbes

### 4-1. Genetic analysis of Acetobacteraceae and Lactobacillacea

Through comparative genomic analysis, amino acid biosynthesis-related genes of *Acetobacteraceae* and *Lactobacillaceae* were compared. As the analysis result, it was confirmed that all enzymes related to branched-chain amino acid (BCAA) biosynthesis were present in *Acetobacteraceae,* whereas *Lactobacillaceae* lacked enzymes related to BCAA biosynthesis (see FIG. 6C). By using the genome of the *A. pomorum* DM001 strain capable of biosynthesis of all amino acids as a template, as the nucleotide sequence similarity of the gene was increased, it was indicated with the blue color, and when the similarity is less than 20%, it was indicated with the red color.

### 4-2. Confirmation of gene deficiencies related to BCAA synthesis in various species of lactic acid bacteria

The following experiment was performed to determine whether BCAA synthesis-related genes were deficient in various lactic acid bacteria other than *Lactobacillus plantarum.*

With respect to *Lactobacillus plantarum* (KCTC14442BP), *Lactobacillus acidophilus* (ATCC 4796), *Lactobacillus casei* (ATCC 393), *Lactobacillus gasseri* (ATCC33323), *Lactobacillus delbrueckii subsp. Bulgaricus* (ATCC 11842), *Lactobacillus helveticus* (ATCC 15009), *Lactobacillus fermentum* (ATCC 14931), *Lactobacillus paracasei* (ATCC 334), *Lactobacillus reuteri* (JCM 1112), *Lactobacillus rhamnosus* (DSMATCC 8530), *Lactobacillus rhamnosus* (DSMATCC 8530), which are 11 species of *Lactobacillus* strains as lactic acid bacteria commonly and broadly used for foods or lactic acid bacteria products (food notification strains), enzymes related to amino acid biosynthesis were identified through comparative genomic analysis. In this regard, the genome of the A. *pomorum* strain capable of biosynthesizing all amino acids was used as a template for comparison (see FIG. 7). As a result of comparative genomic analysis, it was confirmed that all 11 *Lactobacillus* strains were deficient in leuA, leuB, leuC, leuD, ilvA, ilvC, ilvD or ilvE genes related to BCAA biosynthesis (see FIG. 7).

Through these results, it could be found that, due to genetic traits in the case of Lactobacillus plantarum, even if this was introduced into a subject, the nutritional fulfillment effects were not demonstrated.

### 4-3. Proof that the effect of nutrient fulfillment on a subject can be different depending on the genetic traits of the introduced intestinal microbes

In order to clarify that L-EAA deficiency (CNMa hormone expression) in the host can be induced by the genetic trait of the intestinal microbes, *Acetobacteraceae* in which the gene for BCAA synthesis is mutated was prepared as follows, and intestinal deficiency of L-EAA in a host was confirmed. The specific gene mutant strain of *Acetobacter* was produced by amplifying both regions of the gene that is intended to be deleted by PCR, and inserting the same into the pK18mobGII vector, followed by introducing the cloned vector into the wild type *Acetobacter* together with the helper strain (Escherichia coli HB101) in a triparental mating manner.

Specifically, a mutant *Acetobacter* (Aceto ^{ΔleuB}) that cannot biosynthesize leucine among BCAAs was prepared. Drosophila in which the control *Acetobacter* was introduced into sterile Drosophila (+Aceto ^{WT}) showed almost no expression of CNMa hormone. On the other hand, in Drosophila (+Aceto ^{ΔleuB}) introduced with Aceto ^{ΔleuB}, the expression of CNMa hormone was increased to the level of sterile Drosophila.

Further, proC mutant *Acetobacter* (Aceto ^{ΔproC}) that cannot biosynthesize proline as one of the non-essential amino acids, was prepared. Unlike the case where Aceto ^{ΔleuB} was introduced into sterile Drosophila, Drosophila (+Aceto ^{ΔproC}) introduced with Aceto ^{ΔproC} strain did not increase the expression of CNMa hormone (see the left of FIG. 8A).

On the other hand, in Drosophila (+Aceto ^{ΔleuB} +Leucine) obtained by adding leucine to the medium of Drosophila introduced with Aceto ^{ΔleuB}, or in Drosophila (+Aceto ^{ΔleuB_leuB}) introduced with Aceto ^{ΔleuB_leuB} strain which was obtained by genetically reintroducing leuB into Aceto ^{ΔleuB} bacteria, the expression of CNMa hormone was reduced (see the right of FIG. 8A). Similar to the expression pattern of CNMa hormone, it was confirmed that the behavior to fulfill L-EAA nutrition of the host sterile Drosophila was increased when Aceto ^{ΔleuB} was introduced compared to the case where Aceto ^{WT} was introduced into the sterile Drosophila (see FIG. 8B). The behavior of Drosophila introduced with Aceto ^{ΔleuB} to fulfill L-EAA nutrition was reduced when leucine is added to the medium (+Aceto ^{ΔleuB}+Leucine), or when the bacteria genetically reintroduced with deficient leuB are introduced into Drosophila (+Aceto ^{ΔleuB_leuB}) (see FIG. 6B) . This means that the leucine biosynthesis ability of *Acetobacter* plays an important role in the expression of CNMa hormone in intestinal epithelial cells and L-EAA feeding behavior in the host Drosophila.

Similarly, an Aceto ^{ΔilvA} mutant strain that cannot biosynthesize isoleucine among BCAAs was prepared and the same experiment as above was performed (see FIG. 9). As a result of comparing CNMa hormone expression levels of the sterile Drosophila (+None), the control Drosophila (+Aceto ^{WT}) introduced with *Acetobacter* and the Drosophila (+Aceto ^{ΔilvA}) introduced with Aceto ^{ΔilvA} that cannot biosynthesize isoleucine, and the behavior index for L-EAA nutrition fulfillment of the host, the Drosophila (+Aceto ^{ΔilvA}) introduced with Aceto ^{ΔilvA} exhibited CNMa expression level and L-EAA nutrition fulfillment behavior similar to those of sterile Drosophila (+None) (see FIG. 9A). Further, when isoleucine was added to the medium of Drosophila introduced with Aceto ^{ΔilvA} (+Aceto ^{ΔleuB}+Ile) , it was confirmed that CNMa expression level and L-EAA nutrition fulfillment behavior were restored to the level of Drosophila introduced with Aceto ^{WT} (see FIG. 9B).

Further, a gain of function mutant strain capable of BCAA biosynthesis was prepared by introducing 7 enzymes related to BCAA biosynthesis into *Lactobacillus,* which lacks a gene related to BCAA biosynthesis. Specifically, Lacto ^{BCAA} strain having genetically acquired BCAA biosynthesis ability was obtained by amplifying 7 genes for BCAA synthesis such as leuA, leuB, leuC, leuD, ilvA, ilvC and ilvD using the genome of *Lactobacillus coryniformis* subsp. The genome of Torquens DMS 20004 strain was amplified as a template, then inserted it into the PGID023A/B vector, followed by introducing this cloned vector into the Lacto ^{WT} strain through electrophoration and then inserting the same into a specific gene region through homologous recombination.

With respect to the sterile Drosophila (+None), the control *Lactobacillus-introduced* Drosophila (+Lacto ^{WT}), and the Drosophila (+Lacto ^{BCAA}) introduced with the mutant *Lactobacillus* (Lacto ^{BCAA}) having genetically acquired BCAA biosynthesis ability, the CNMa hormone expression level and L-EAA nutrition fulfillment behavior were observed. As a result, it was confirmed that the Drosophila introduced with Lacto ^{BCAA} had reduced CNMa hormone expression and L-EAA nutrition fulfillment behavior index, compared to the Drosophila introduced with Lacto ^{WT} (see FIG. 10A). Further, when BCAA (isoleucine, leucine, valine) was added to the medium of Drosophila introduced with Lacto ^{WT}, it was confirmed that CNMa expression and L-EAA nutrition fulfillment behavior index were decreased to the level of Drosophila introduced with Lacto ^{BCAA} (see FIG. 10B).

Through the above results, it was confirmed that the deficiency of L-EAA in the host was offset or not offset depending on the genetic traits of the intestinal microbes in the nutrient deficient situation of the host animal. In particular, in the case of *Lactobacillus* as a representative lactic acid bacterium, it was difficult to synthesize BCAAs, such that it could not offset the deficiency of L-EAA in the host gut, and thereby showing induction of the feeding preference to fulfill the host's nutrition. Further, it was confirmed that, if BCAA is supplemented, hormonal changes and behavioral changes (preference index) of the host caused by lactic acid bacteria disappeared.

### 5. Growth promotion and intestinal damage improvement effect of lactic acid bacteria and BCAA combination in nutrient-deficient subjects

Through the above-described experimental results, even if a nutrient-deficient subject takes lactic acid bacteria, it will be difficult to supplement nutrients. Further, it was demonstrated that, if BCAAs, which are not synthesized well in lactic acid bacteria by intake of lactic acid bacteria, are ingested in combination, the nutritional needs of the subject will be fulfilled. Based on these results, it is believed that treatment of BCAA together with the lactic acid bacteria can enhance the effect of lactic acid bacteria alone. Therefore, with respect to Drosophila animal model and mouse animal model, it was investigated whether the lactic acid bacteria can enhance physiologically active efficacy of the host (the subject to which lactic acid bacteria are introduced) by supplementing BCAA together with the lactic acid bacterial in the nutritionally deficient situation.

### 5-1. Confirmation of growth-promoting effect of combination treatment of lactic acid bacteria and BCAA on nutrient-deficient subjects

### 5-1-1. Combination effect of lactic acid bacteria with three BCAAs including leucine, isoleucine and valine

With respect to the sterile Drosophila model, growth changes were observed by adding *Lactobacillus* and BCAA (including all of leucine, isoleucine and valine) separately or together to the sterile Drosophila in a protein-deficient medium (1% yeast, 10% sucrose, cornmeal medium). Specifically, for a group in which BCAAs (leucine, isoleucine and valine) were added to sterile Drosophila EGG (+BCAA); a group introduced with *L. plantarum* WJL alone (+WJL); and a group (+WJL+BCAA) in which *L. plantarum* WJL bacteria and BCAAs (leucine, isoleucine and valine) were introduced together, pupae formation was observed at an interval of 12 hours. Then, an average period required for development (hereinafter referred to as an average development duration) was measured.

As a result, the BCAA (leucine, isoleucine and valine alone addition) group had the average development duration similar to that of the control group (+None). This means that supplementation of nutrients alone cannot compensate for the lack of growth in the nutritionally deficient situation. The only lactic acid bacteria-introduced group had a shorter average duration than the control group and the BCAA alone addition group. However, it was confirmed that the group introduced with lactic acid bacteria together with BCAA showed a statistically significant increase in the growth promoting efficacy (see FIG. 11, *** (p < 0.001), ** (P < 0.01), * (P < 0.05), ns: not statistically significant).

### 5-1-2. Combination effect of lactic acid bacteria and at least two BCAAs among leucine, isoleucine and valine

Further, the following experiment was performed in order to investigate whether the growth promoting efficacy appears even if leucine, isoleucine or valine alone is used in combination with the lactic acid bacteria, or other amino acids are used in combination with the lactic acid bacteria in nutrient-deficient subjects. The same procedures as the above-described experimental method of 5-5-1 were conducted except that different types of amino acid were treated to observe the growth change of nutrient-deficient Drosophila subjects.

As a result, similar to 5-5-1, the groups in which only amino acids were treated and lactic acid bacteria were not treated (None+3BCAA (=BCAA); None+Leucine; None+Isoleucine; None+Valine; None+Histidine; None+Leucine+Valine; None+Leucine+Isoleucine; None+Isoleucine+Valine) had the average development duration similar to those of the untreated group (None+None) (the left of FIGS. 12A and 12B).

On the other hand, the groups treated using lactic acid bacteria together with at least two or more of leucine, isoleucine and valine (WJL+BCAA; WJL+Leucine+Valine; WJL+Leucine+Isoleucine; WJL+Isoleucine+Valine) showed statistically shortened average development duration, as compared to the group treated with lactic acid bacteria or amino acids alone (the right of FIG. 12B). Further, the groups, in which leucine, isoleucine or valine alone was treated together with lactic acid bacteria (WJL+Leucine; WJL+Isoleucine; WJL+Valine), did not show statistically significant difference in the average development duration as compared to the group treated with lactic acid bacteria alone (the right of FIG. 12A).

Through the above results, it can be confirmed that the present invention does not simply use amino acids in combination for nutritional supplementation, instead, two or more types of BCAA amino acids act on lactic acid bacteria and thus can serve to enhance the activity of lactic acid bacteria in subjects.

### 5-1-3. Combination effect of BCAAs and various lactic acid bacteria

It was confirmed whether other species of lactic acid bacteria other than *Lactobacillus plantarum* strain can also enhance growth promoting efficacy by BCAAs (leucine, isoleucine and valine) in nutrient-deficient subjects.

The same procedures as the above-described experimental method of 5-5-1 were executed except that 11 types of food notification strains belonging to the Lactobacillus group that were confirmed to be deficient in BCAA biosynthesis through genome comparison (3 species of *Lactobacillus plantarum* (WJL, Nizo and Nc8); 2 species of *Lactobacillus paracasei* (KCTC5058 and IH30-12); 3 species of *Lactobacillus rhamnosus* (KCTC3237, GG and IH37-25); 2 species of *Lactobacillus fermentum* (KCTC5467 and IH37-57); 3 species of *Lactobacillus casei* (IH37-55, IH37-56 and IH37-9); 1 species of *Lactobacillus acidophilus* (KCTC3594); 1 species of *Lactobacillus gasseri* (KCTC3143); 2 species of Lactobacillus vulgaricus (IH37-37 and IH37-19); 1 species of Lactobacillus reuteri (KCTC3594); 2 species of *Lactobacillus helveticus* (KCTC3545 and KCTC15060); 2 species of *Lactobacillus salivarius* (KCTC3157 and IH37-38)) were used.

As a result, it was confirmed that other species of lactic acid bacteria other than *Lactobacillus plantarum* also showed significant improvement of the growth promoting efficacy by treatment of the lactic acid bacteria in combination with BCAA (see FIGS. 13 and 14).

### 5-2. Confirmation of growth promoting effect and intestinal damage improvement effect when lactic acid bacteria and BCAA are combined and treated to nutrient-deficient subjects

Based on the fact that growth may be inhibited or intestinal damage may occur in nutrient-deficient subjects, it was investigated whether the effects of promoting growth and inhibiting intestinal damage appear in a mouse model when treated with a combination of lactic acid bacteria and BCAA.

Specifically, a three-week-old mouse animal model after completion of weaning was fed with insufficient nutrients, followed by identifying whether there is growth and intestinal damage improvement after treatment with lactic acid bacteria and BCAAs (leucine, isoleucine and valine) alone or in combination: control (None); L. *plantarum* WJL alone treatment group (+WJL); and *L. plantarum* WJL and BCAA (leucine, isoleucine and valine) combination treatment group (+WJL+BCAA). Four to five mice were set per group, food was supplied daily, and indexes such as body weight were tracked every two days. After 12 weeks of observation, the mice were dissected to monitor the leaky gut (FITC), the degree of bone growth, the bone mineral density, and other disease indexes of other organs. In order to measure the leaky gut, a fluorescent substance such as fluorescein isothiocyanate-dextran (FITC-dextran, sigma #FD4) was used and the same amount of FITC-dextran was fed to each mouse, followed by measuring FITC fluorescence in the blood 4 hours later. In order to measure the bone density of mice, the femurs of mice were dissected and sampled, followed by transferring and storing the same in 70% ethanol solution at 4 °C. For use of a Micro-CT device (SkyScan 1276, Bruker), the bones were transferred from 70% ethanol solution to tertiary distilled water 48 hours before imaging and then stored at 4 °C. Bone images were taken using the SkyScan 1276 program. When shooting, the voxel size was fixed at 32 um and the pictures were taken under the conditions of 70 kV and 57 µA. The NRecon program was used to reconstruct the captured image in 3D, and the CTAn program was used to measure the bone mineral density (BMD), bone length, and cortical bone thickness using the 3D reconstructed image. For BMD measurement, two bone density phantoms with densities of 0.25 g/cm³ and 0.75 g/cm³, respectively, were used to calculate BMD proportional equation, followed by designating and measuring the mid-diaphysis part of the femur. By dragging and designating the entire femur, the actual bone length was measured according to the calculation method inherent in the program. Cortical bone thickness was measured in the central section of the mid-diaphysis part of the femur.

As a result, in the mouse group treated with both lactic acid bacteria and BCAAs (leucine, isoleucine and valine), a statistically significant weight gain effect and a leaky gut inhibitory effect were observed (see FIG. 15). Further, in the case where the lactic acid bacteria and BCAAs (leucine, isoleucine and valine) were introduced together, the bone density was statistically and significantly increased compared to the case where each of the above compounds was treated alone (see FIG. 16). On the other hand, as shown in FIG. 16, even if the lactic acid bacteria were treated alone, it could be seen that the bone density was increased, therefore, the lactic acid bacteria itself had a growth promoting effect. However, it was confirmed that BCAA significantly increased the growth promoting effect of these lactic acid bacteria.

Through the above experiments, the growth of nutrient-deficient subjects was promoted by treating at least two or more of leucine, isoleucine and valine in combination with the lactic acid bacteria rather than when lactic acid bacteria alone or BCAA, in particular, the lactic acid bacteria were treated alone. Further, it was also confirmed that a disease-associated index was improved.

### [The Deposit of Microorganism]

### RECEIPTE IN THE CASE OF AN ORIGINAL DEPOSIT

Issued pursuant to Rule 7.1 of Budapest Treaty on the International Recognition of the Deposit of Microorganisms for the Purposes of Patent Procedure

To: Seoul National University Industry-University Institute Collaboration
1 Gwanak-ro, Gwanak-gu, Seoul, Korea

| I. IDENTIFICATION OF THE MICROORGANISM | |
|---|---|
| Identification reference given by the DEPOSITOR: | Accession number given by the INTERNATIONAL DEPOSITARY AUTHORITY: |
| **Lactobacillus plantarum WJL** | **KCTC 14442 BP** |

| II. SCIENTIFIC DESCRIPTION AND/OR PROPOSED TAXONOMIC DESIGNATION | |
|---|---|
| The microorganism identified under I above was accompanied by: | |
| ( ) a scientific description | |
| ( ) a proposed taxonomic designation | |
| (Mark with a cross where applicable) | |

| III. RECEIPT AND ACCEPTANCE | |
|---|---|
| This International Depositary Authority accepts the microorganism identified under I above, which was received | |
| by January 11, 2021. | |

| IV. RECEIPT OF REQUEST FOR CONVENRSION | |
|---|---|
| The microorganism identified under I above was received by this International Depositary Authority on (date of the original deposit) and a request to convert the original deposit to a deposit under the Budapest Treaty was received by it on (date of receipt of request for conversion). | |

| V. INTERNATIONAL DEPOSITARY AUTHORITY | |
|---|---|
| Name: Korean Collection for Type Cultures (KCTC) | Signature (s) of person (s) having the power to represent the International Depositary Authority or of authorized official (s) : |
| Address: Korea Research Institute of Bioscience and Biotechnology (KRIBB) Ipsin-gil, Jeongeup-shi, Jeolla-bukdo, Korea (56212) | |
| | Date: January 11, 2021 |

Form BP/4 (KCTC Form 17)

## Claims

1. A composition for enhancing efficacy of lactic acid bacteria, comprising: at least two branched-chain amino acids (BCAAs) selected from the group consisting of leucine, isoleucine and valine.

2. The composition according to claim 1, comprising leucine, isoleucine and valine.

3. The composition according to claim 1, wherein the efficacy of lactic acid bacteria is to promote the growth of a subject or to inhibit intestinal damage.

4. The composition according to claim 1, wherein the efficacy of lactic acid bacteria is to increase at least one of body weight or bone density of a nutrient-deficient subject, or to inhibit leaky gut of the nutrient-deficient subject.

5. The composition according to claim 1, wherein the lactic acid bacteria is at least one selected from the group consisting of *Lactobacillus spp., Lactococcus spp., Enterococcus spp., Streptococcus spp.* and *Bififobacterium spp.*

6. The composition according to claim 5, wherein the *Lactobacillus spp.* is at least one selected from the group consisting of: *Lactiplantibacillus plantarum; Lactobacillus paracasei; Lactobacillus rhamnosus; Lactobacillus fermentum; Lactobacillus casei; Lactobacillus acidophilus; Lactobacillus gasseri; Lactobacillus delbrueckii subsp. Bulgaricus; Lactobacillus reuteri; Lactobacillus helveticus;* and *Lactobacillus salivarius.*

7. A pharmaceutical composition for promoting growth or inhibiting intestinal damage, comprising: the composition according to any one of claims 1 to 6; and lactic acid bacteria.

8. The pharmaceutical composition according to claim 7, wherein the lactic acid bacteria is at least one selected from the group consisting of: *Lactiplantibacillus plantarum; Lactobacillus paracasei; Lactobacillus rhamnosus; Lactobacillus fermentum; Lactobacillus casei; Lactobacillus acidophilus; Lactobacillus gasseri; Lactobacillus delbrueckii subsp. Bulgaricus; Lactobacillus reuteri; Lactobacillus helveticus;* and *Lactobacillus salivarius.*

9. A pharmaceutical composition for preventing or treating at least one disease selected from the group consisting of growth disorders, hypogrowth, osteoporosis, osteomalacia, osteopenia, environmental enteropathy and leaky gut syndrome, comprising: the composition according to any one of claims 1 to 6; and lactic acid bacteria.

10. A food composition for promoting growth or inhibiting intestinal damage, comprising: the composition according to any one of claims 1 to 6; and lactic acid bacteria.
